# FASCICULE DE BREVET EUROPEEN

(11) **EP 1 795 137 B1**
(45) Date de publication et mention de la délivrance du brevet: **04.03.2009**
(21) Numéro de dépôt: 06356139.3
(22) Date de dépôt: 06.12.2006
(51) Int. Cl.: A61B 17/70

(54) **Dispositif de stabilisation latérale du rachis**
Einrichtung zur lateralen Stabilisierung der Wirbelsäule
Apparatus for lateral stabilisation of the spine

(30) Priorité: 07.12.2005 FR 0512426
(43) Date de publication de la demande: 13.06.2007
(73) Titulaire: PHUSIS, 38330 Saint Ismier (FR)
(72) Inventeur: Tornier, Alain, 38330 St. Ismier (FR); Ferrari, épouse Gosset, Irène, 38660 Saint Vincent de Mercuze (FR)
(74) Mandataire: Myon, Gérard Jean-Pierre

(56) Documents cités:
- DE-U1- 29 814 320
- US-A- 5 423 816
- US-A1- 2005 113 927
- US-B1- 6 296 644
- PATENT ABSTRACTS OF JAPAN vol. 1999, no. 01, 29 janvier 1999 (1999-01-29) & JP 10 277070 A (SANO SHIGEO; ITO KAORU), 20 octobre 1998 (1998-10-20)

## Description

La présente invention un dispositif de stabilisation latérale du rachis, destiné à être implanté le long de la colonne vertébrale, au niveau d'un ou des deux de ses côtés latéraux gauche et droit, en vue de stabiliser au moins deux vertèbres l'une par rapport à l'autre. Une telle stabilisation dynamique est recherchée notamment dans le cadre du traitement du rachis dégénératif ou traumatique. L'invention s'intéresse plus particulièrement au traitement du rachis dorso-lombaire mais s'applique également au traitement du rachis cervical.

Pour traiter une instabilité intervertébrale, une première possibilité connue consiste à fusionner deux vertèbres adjacentes, ce qui revient à priver ces deux vertèbres de leur liberté de mouvements relatifs. Des montages totalement rigides sont à cet effet implantés de manière fixe le long du rachis pour bloquer définitivement la liaison articulaire entre les deux vertèbres à fusionner. US-B-6,296,644 propose ainsi un montage vertébral constitué de plusieurs éléments vertébraux qui sont chacun à fixer sur autant de vertèbres et qui sont reliés deux à deux par des liaisons « blocables » : lors de la mise en place du montage, ces liaisons sont mobiles, pour accommoder le positionnement relatif des éléments vertébraux le long du rachis, puis, à la fin de cette mise en place, les liaisons sont figées à demeure par des bagues à mémoire de forme, de sorte que, en service, les éléments vertébraux sont complètement fixes les uns par rapport aux autres. Ce genre d'intervention d'arthrodèse des vertèbres conduit cependant à une dégénérescence des disques adjacents sur lesquels il est nécessaire d'intervenir ultérieurement.

Une autre possibilité connue de traitement du rachis consiste à intervenir à un stade plus précoce que celui impliquant une arthrodèse. Une première solution de ce type est proposée dans DE-U-298 14 320 : plusieurs éléments vertébraux distincts, respectivement fixés à des vertèbres adjacentes, sont en service mobiles les uns par rapport aux autres, en étant reliés deux à deux par des liaisons télescopiques rectilignes suivant la direction longitudinale du rachis. Ce montage mobile s'accommode d'une certaine évolution du comportement cinématique du rachis, par exemple lors de sa croissance, mais n'assure pas une réelle stabilisation dynamique des vertèbres, n'empêchant ainsi pas, par exemple, l'écrasement ou la déformation des disques intervertébraux.

Une deuxième solution est proposée dans JP-A-10 277070 : deux pistons relient verticalement les côtés postérieurs de deux vertèbres adjacentes, avec interposition, entre la partie mâle et la partie femelle de chaque piston, d'une chemise en matériau élastique. Chaque piston présente une section transversale elliptique, ce qui centre les mouvements d'articulation entre les parties mâle et femelle de chaque piston soit dans le piston, soit dans un plan passant par les deux pistons, c'est-à-dire bien en arrière des vertèbres. La cinématique imposée aux vertèbres est ainsi très éloignée des comportements anatomiques normaux du rachis, avec des risques importants que le disque intervertébral se retrouve pincé, voire écrasé au moins dans sa portion antérieure.

D'autre solutions visent à implanter un dispositif latéral de stabilisation dynamique, tel que ceux proposés dans US-A-5,423,816, US-A-5,704,936 et US-B-6,616,669. A cet effet, ce genre de dispositif comporte, d'une part, des éléments rigides à ancrer dans l'os d'un même côté latéral de deux vertèbres adjacentes et, d'autre part, des éléments souples de liaison entre ces éléments rigides. Ces éléments souples, tels que des ressorts ou des bras flexibles, s'étendent latéralement le long du rachis et soulagent ainsi le disque intervertébral en réduisant d'éventuelles surpressions au niveau des surfaces articulaires entre ce disque et les vertèbres. Ces dispositifs offrent plus de confort au patient car ils permettent de conserver la mobilité du rachis. Cependant, en pratique, l'utilisation de ce genre de dispositif dynamique se révèle délicate. Le dimensionnement de la flexibilité des éléments de liaison est difficile puisqu'il doit être adapté à chaque patient selon sa pathologie et sa morphologie et, à la longue, les comportements élastiques de ces éléments évoluent. Le mauvais contrôle de ces paramètres ne permet pas de garantir le respect d'une cinématique appropriée au rachis, ce qui peut conduire à une mauvaise stabilisation de l'écartement intervertébral et à une aggravation des lésions que l'on cherche à traiter.

Le but de la présente invention est de proposer un dispositif de stabilisation latérale du rachis reproduisant plus fidèlement les mouvements anatomiques des vertèbres, plus efficace pour stabiliser les vertèbres traiter et plus fiable dans le temps.

A cet effet, l'invention a pour objet un dispositif de stabilisation latérale du rachis destiné, en service, à reproduire une liaison intervertébrale articulaire, tel que défini à la revendication 1.

Le fait de guider les deux éléments vertébraux l'un par rapport à l'autre par des surfaces portées par ces éléments et coopérant par complémentarité de formes permet de conférer au dispositif un comportement cinématique précis et stable dans le temps. La cinématique imposée, à savoir un basculement relatif entre les deux éléments autour d'un axe médio-latéral ou à peu près médio-latéral, garantit que les mouvements articulaires intervertébraux provoqués par une sollicitation du rachis, notamment en flexion/extension, sont efficacement centrés autour d'un axe précis dont la position prédéterminée dans l'espace discal est prévue pour que ces comportements soient quasi-identiques ou, tout au moins, les plus proches possibles des comportements anatomiques normaux du rachis. De la sorte, la coopération de ces surfaces de guidage en appui l'une sur l'autre peut permettre de conserver un espacement intervertébral satisfaisant, en maintenant un écartement vertical des vertèbres prédéterminé. Le dispositif selon l'invention supporte ainsi l'essentiel, voire la totalité des contraintes appliquées au disque intervertébral, ce dernier demeurant mobile. En outre, l'implantation du dispositif selon l'invention se révèle particulièrement facile puisque les mobilités internes au dispositif résident essentiellement, voir exclusivement, au niveau des surfaces de guidage portées par les deux éléments vertébraux dont les positions d'ancrage dans les deux vertèbres à traiter sont choisies et fixées par le chirurgien.

D'autres caractéristiques avantageuses de ce dispositif, prises isolément ou suivant toutes les combinaisons techniquement possibles, sont énoncées aux revendications dépendantes 2 à 11.

L'invention sera mieux comprise à la lecture de la description qui va suivre, donnée uniquement à titre d'exemple et faite en se référant aux dessins sur lesquels :
- la figure 1 est une vue en perspective de deux vertèbres adjacentes équipées d'un dispositif de stabilisation latérale selon l'invention, ces vertèbres et ce dispositif étant observés par l'arrière et de manière décalée par rapport au plan sagittal du rachis, en étant représentés avec un écartement intervertébral supérieur à la normale pour des raisons de visibilité ;
- la figure 2 est une vue en élévation, prise suivant la flèche II de la figure 1, des moitiés latérales des vertèbres et du dispositif, la figure 2 correspondant ainsi à une vue par l'avant du rachis ;
- les figures 3 et 4 sont respectivement des sections prises suivant les lignes III-III de la figure 2 et IV-IV de la figure 3 ; et
- la figure 5 est une section analogue à la figure 4, d'une variante du dispositif selon l'invention.

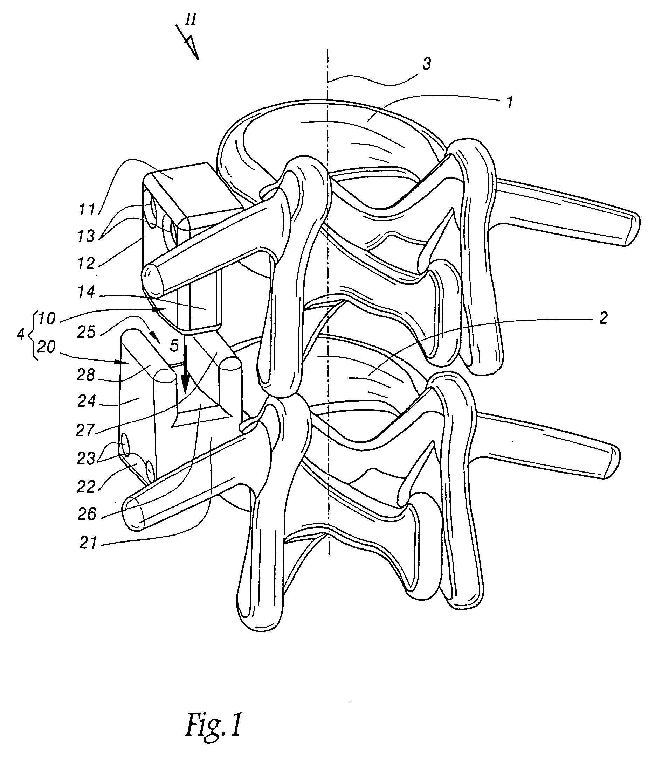

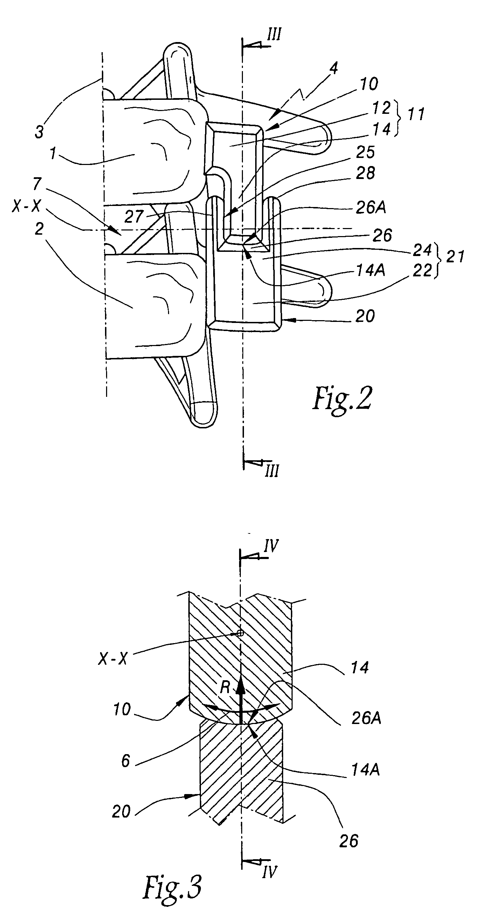

Sur les figures 1 et 2 sont représentées deux vertèbres adjacentes 1 et 2 d'un rachis lombaire d'un être humain. La direction longitudinale de ce rachis est référencée 3, les vertèbres 1 et 2 étant séparées l'une de l'autre, suivant cette direction, par un disque intervertébral non représenté sur les figures pour des raisons de visibilité. Par commodité, la suite de la description est orientée par rapport à ces vertèbres dans leur position anatomique, c'est-à-dire que les termes « postérieur » ou « arrière », « antérieur » ou « avant », « droit », « gauche », « supérieur », « inférieur », etc. s'entendent par rapport au rachis du patient se tenant debout. De même, le terme « sagittal » correspond à une direction dans le sens antéro-postérieur, verticalement sur la ligne médiane du rachis, tandis que le terme « médial » correspond à une direction sensiblement perpendiculaire au plan sagittal du rachis, dirigée vers ce rachis, le terme « latéral » correspondant à une direction de sens opposé.

Sur les figures 1 à 4 est représenté un dispositif 4 de stabilisation dynamique des vertèbres 1 et 2, implanté sur le côté gauche des vertèbres, en vue de reproduire la liaison articulaire entre ces vertèbres, notamment lorsque ces dernières sont sollicitées en flexion/extension, tout en assurant un espacement intervertébral satisfaisant. Ce dispositif comporte un élément vertébral supérieur 10 implanté au niveau de la vertèbre 1 et un ensemble vertébral inférieur 20 implanté au niveau de la vertèbre 2.

Chaque élément vertébral 10, 20 comporte un corps rigide 11, 21 d'une seule pièce, par exemple métallique, adapté pour être fixé au côté gauche de la vertèbre 1, 2. A cet effet, la partie supérieure 12 du corps 11 et la partie inférieure 22 du corps 21 sont traversées de part en part, suivant une direction médio-latérale, par des orifices 13, 23 destinés à recevoir des vis, non représentées, d'ancrage osseux dans le corps vertébral des vertèbres 1 et 2 pour immobiliser fermement les éléments vertébraux 10 et 20 par rapport aux vertèbres.

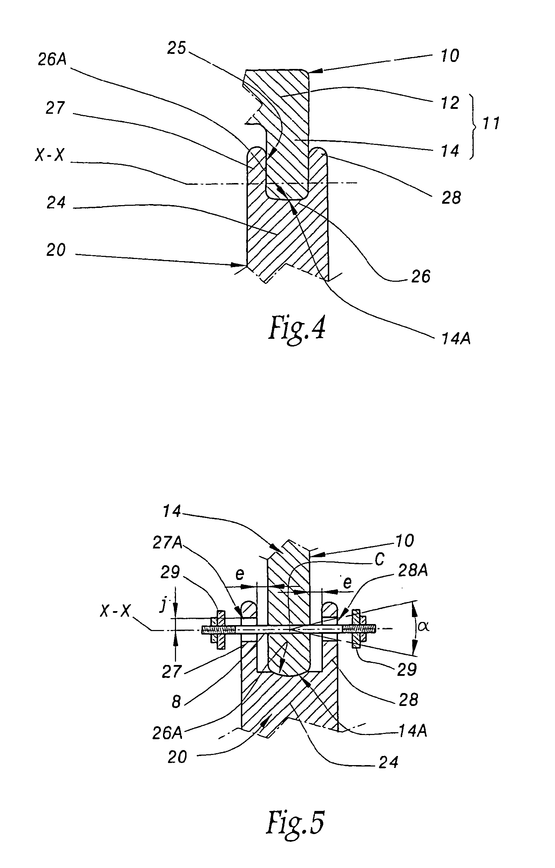

La partie inférieure 14 de l'élément 10 et la partie supérieure 24 de l'élément 20 sont adaptées pour coopérer l'une avec l'autre lorsque le dispositif 4 est implanté, comme représenté sur la figure 2. En coupe frontale, comme à la figure 4, c'est-à-dire dans un plan de coupe sensiblement vertical et parallèle à une direction médio-latérale, le corps 11, constitué des parties supérieure 12 et inférieure 14 venues de manière l'une avec l'autre, présente une section en forme de L retourné tandis que la partie supérieure 24 présente une section globalement en forme de U. Cette partie supérieure 24 définit ainsi un logement 25 débouchant librement vers le haut et destiné à recevoir la partie inférieure 14. Ce logement 25 est délimité inférieurement par une paroi de fond 26 et, sur ses côtés, par une paroi médiale 27 et une paroi latérale 28 parallèles l'une à l'autre, ces parois 26, 27 et 28 étant venues de matière avec le reste du corps 21.

L'écartement médio-latéral entre les parois 27 et 28 est sensiblement égal à la dimension médio-latérale de la partie inférieure 14 de l'élément 10 de sorte que, lorsque cette partie est reçue dans le logement 25 comme indiqué par la flèche 5 à la figure 1, elle est retenue suivant une direction médio-latérale, sans possibilité de débattement, à des jeux fonctionnels près, comme représenté sur les figures 2 et 4.

A la différence des surfaces en regard des parois médiale 27 et latérale 28, la surface supérieure 26A de la paroi de fond 26 n'est pas plane mais est bombée avec sa concavité tournée vers le haut. Plus précisément, cette surface 26A correspond à une portion de cylindre d'axe X-X s'étendant au-dessus de la paroi de fond 26 et suivant une direction sensiblement médio-latérale, comme représenté aux figures 2 à 4. Cette surface 26A est adaptée pour guider les mouvements relatifs entre les éléments supérieur 10 et inférieur 20 du dispositif 4, en coopérant par complémentarité de formes avec la surface d'extrémité inférieure 14A de la partie 14 de l'élément 10, cette surface 14A correspondant, elle aussi, à une portion de cylindre d'axe sensiblement confondu avec l'X-X lorsque les éléments sont implantés. Autrement dit, lorsque les éléments 10 et 20 sont fixés aux vertèbres 1 et 2, les surfaces 14A et 26A sont appuyées l'une contre l'autre, de manière basculante autour de l'axe X-X, comme indiqué par la flèche 6 à la figure 3.

Pour ne pas gêner les mouvements relatifs de basculement entre les éléments 10 et 20, les côtés antérieur et postérieur du logement 25 débouchent librement sur l'extérieur.

En service, lorsque le dispositif 4 est implanté sur les vertèbres 1 et 2, les surfaces 14A et 26A coopèrent de manière à guider le basculement entre les vertèbres autour de l'axe X-X. Comme cet axe s'étend avantageusement dans l'espace intervertébral 7 séparant les vertèbres 1 et 2 suivant la direction longitudinale 3 du rachis, en particulier dans la région médiane de cet espace, les mouvements de basculement imposés par la coopération de ces surfaces sont identiques aux ou, tout au moins, très proches des mouvements articulaires intervertébraux anatomiques générés lors d'une flexion ou d'une extension du rachis.

L'implantation latérale du dispositif 4 est particulièrement aisée et rapide puisque seuls les éléments 10 et 20 sont à rapportés fermement aux vertèbres 1 et 2, au moyen des vis d'ancrage osseux précitées. En pratique, les éléments 10 et 20 sont mis en place simultanément, avec la partie inférieure 14 de l'élément 10 reçue dans le logement supérieur 25 de l'élément 20, comme indiqué par la flèche 5 à la figure 1. De la sorte, le chirurgien peut implanter le dispositif 4 avec les surfaces 14A et 26A au contact l'une de l'autre et pour une configuration d'extension prédéterminée des vertèbres. Le dispositif 4 maintient ainsi l'écartement longitudinal des vertèbres 1 et 2, suivant la direction 3, sous une contrainte prédéterminée par le chirurgien. On comprend que, à cet effet, les surfaces 14A et 26A doivent globalement s'étendre selon une direction antéro-postérieure et coopérer ainsi de manière pressante dans la direction longitudinale 3 du rachis. Autrement dit, les surfaces 14A et 26A coopèrent l'une avec l'autre en formant une résultante de contact R s'étendant sensiblement parallèlement à cette direction 3.

Sur la figure 5 est représentée une variante du dispositif 4, destinée à permettre au dispositif de présenter une légère liberté de débattements internes suivant des directions transversales à une stricte direction médio-latérale, assurant un plus grand confort au patient lors de mouvements de torsion de son rachis, c'est-à-dire de rotation autour de la direction longitudinale du rachis, et/ou d'inclinaison latérale de son rachis. A cet effet, cette variante se distingue du dispositif des figures 1 à 4 par la géométrie des surfaces de guidage coopérantes 14A et 26A : dans cette variante, ces surfaces correspondent sensiblement à une même portion de sphère centrée en un point C situé sur un axe médio-latéral X-X. En principe, les éléments 10 et 20 sont ainsi articulés l'un par rapport à l'autre à la façon d'une rotule de centre C. En pratique, seul le mouvement de basculement autour de l'axe X-X est librement possible, les autres mouvements admissibles étant limités par une tige 8 d'axe longitudinal correspondant à l'axe X-X, qui traverse librement et de part en part la partie inférieure 14 de l'élément 10. Les deux extrémités longitudinales de cette tige 8 sont reçues, avec un jeu de débattement j, dans respectivement des orifices 27A et 28A cylindriques à base circulaire, ménagées à travers les parois médiane 27 et latérale 28 de l'élément 20. Le diamètre des extrémités de la tige 8 est inférieur au diamètre de ces orifices 27A et 28A de manière à définir le jeu de débattement j. De la sorte, les mouvements rotatifs autour du point C entre les éléments 10 et 20, autre que le mouvement de basculement autour de l'axe X-X, ne sont autorisés que jusqu'à ce que les extrémités de la tige 8 butent dans l'une des parois définissant les orifices 27A et 28A.

Pour des raisons de maintien mécanique, des rondelles 30 sont respectivement rapportées autour de chaque extrémité de la tige 8, respectivement en appui contre le côté médial de la paroi 27 et contre le côté latéral de la paroi 28. Chaque rondelle est associée à un écrou de blocage 32, par exemple vissé autour de l'extrémité libre filetée de l'extrémité correspondante de la tige 8.

Pour éviter que les parois médiale 27 et latérale 28 ne constituent une gêne à ces mouvements de débattement rotatifs entre les éléments 10 et 20, un écartement médio-latéral e non nul est prévu entre, d'une part, la paroi 27 et la face médiale de la partie 14 et, d'autre part, entre la paroi 28 et la face latérale de la partie 14. En outre, les rondelles de retenue 29 montées sur la tige 8 sont disposées à distance des parois 27 et 28.

En variante, au lieu d'être disposées à distance des parois 27 et 28, les rondelles 29 pourraient être en portion de sphère centrée sur le centre C, les faces externes des parois 27 et 28 ayant la même géométrie.

On remarquera que, contrairement au dispositif des figures 1 à 4, dans lesquelles l'axe de basculement médio-latéral X-X est exclusivement défini par la coopération des surfaces 14A et 26A, l'axe de basculement X-X entre les éléments 10 et 20 de la variante du dispositif de la figure 5 est défini, à la fois, par la coopération des surfaces sphériques 14A et 26A et par la présence de la tige 8 matérialisant cet axe. En pratique, la direction de l'axe de basculement X-X pour le dispositif des figures 1 à 4 est imposé de manière sensiblement confondue avec une direction médio-latérale vis-à-vis du rachis tandis que, avec le dispositif de la figure 5, cet axe de basculement X-X peut, au cours de la sollicitation du rachis, être incliné par rapport à la direction médio-latérale au rachis, l'angle maximal α de cette inclinaison étant toutefois limité à quelques degrés seulement, notamment à une dizaine de degrés.

Divers aménagements et variantes au dispositif 4 évoqué ci-dessus sont en outre envisageables :
- dans l'exemple de réalisation détaillé ci-dessus, l'axe X-X de basculement entre les éléments 10 et 20 est un axe géométrique ; en variante, on peut prévoir qu'un axe physique s'étend à travers le logement 25 et relie les parois médiale 27 et latérale 28, en étant reçu dans un orifice complémentaire traversant de part en part la partie inférieure 14 de l'élément 10, suivant une direction médio-latérale ;
- les courbures des surfaces de guidage 14A et 26A peuvent être inversées ;
- la structure mâle/femelle du dispositif 4 peut être inversée de sorte que, en variante, l'élément vertébral supérieur définit un logement inférieur, analogue au logement 25, à l'intérieur duquel est reçue la partie supérieure complémentaire de l'élément vertébral inférieur ;
- plutôt que de prévoir les surfaces 14A et 26A rigoureusement cylindriques, de sorte qu'elles basculent en glissant l'une contre l'autre autour de l'unique axe X-X, ces surfaces peuvent présenter des profils bombés définissant, lors du basculement relatif entre les éléments 10 et 20, une pluralité d'axes instantanés de rotation, parallèles les uns aux autres et s'étendant suivant des directions médio-latérales ; et/ou
- dans l'exemple de réalisation représenté aux figures, le dispositif 4 n'est implanté qu'au niveau du côté gauche des vertèbres 1 et 2 ; en variante, le dispositif comprend, en remplacement des éléments 10 et 20, deux éléments vertébraux analogues aux éléments 10 et 20 et adaptés pour être implantés sur le côté droit des vertèbres ; de même, le dispositif selon l'invention peut comprendre quatre élément vertébraux associés par paires, respectivement prévues sur les côtés gauche et droit des vertèbres ; dans ce cas, pour homogénéiser les comportements cinématiques de ces deux dispositifs, on peut prévoir qu'un axe physique relie les deux paires d'éléments, cet axe s'étendant suivant la direction X-X et constituant un axe de basculement commun pour les deux paires d'éléments, en traversant le disque intervertébral suivant une direction médio-latérale.

## Revendications

1. Dispositif (4) de stabilisation latérale du rachis destiné, en service, à reproduire une liaison intervertébrale articulaire, comportant au moins deux éléments vertébraux (10, 20) respectivement adaptés pour être fixés sur un même côté latéral du corps d'au moins deux vertèbres adjacentes (1, 2), **caractérisé en ce que** les deux éléments vertébraux (10, 20) délimitent des surfaces respectives (14A, 26A) de guidage relatif des deux éléments, adaptées pour, lorsque les éléments sont implantés sur leur vertèbre correspondante, coopérer l'une avec l'autre par complémentarité de formes de telle sorte que ces surfaces définissent un axe de rotation (X-X) autour duquel les deux éléments peuvent basculer l'un par rapport à l'autre et qui s'étend, à la fois, suivant une direction essentiellement médio-latérale au rachis et dans l'espace discal intervertébral (7) séparant les deux vertèbres adjacentes.

2. Dispositif suivant la revendication 1, **caractérisé en ce que** les surfaces de guidage (14A, 26A) définissent un axe permanent de rotation (X-X) lorsque les vertèbres (1, 2) équipées des deux éléments vertébraux (10, 20) sont déplacées l'une par rapport à l'autre selon un mouvement de flexion-extension du rachis.

3. Dispositif suivant la revendication 1, **caractérisé en ce que** les surfaces du guidage (14A, 26A) définissent plusieurs axes instantanés de rotation lorsque les vertèbres (1, 2) équipées des deux éléments vertébraux (10, 20) sont déplacées l'une par rapport à l'autre selon un mouvement de flexion-extension du rachis.

4. Dispositif suivant l'une quelconque des revendications précédentes, **caractérisé en ce que** les surfaces de guidage (14A, 26A) sont également adaptées pour maintenir un écartement minimal entre les vertèbres (1, 2) suivant la direction longitudinale (3) du rachis.

5. Dispositif suivant la revendication 4, **caractérisé en ce que** la résultante (R) de contact des deux surfaces de guidage (14A, 26A) s'étend sensiblement parallèlement à la direction longitudinale (3) du rachis.

6. Dispositif suivant l'une quelconque des revendications précédentes, **caractérisé en ce que** les surfaces de guidage (14A, 26A) sont respectivement ménagées par une partie mâle (14) de l'un (10) des deux éléments vertébraux (10, 20) et par une partie femelle (24) de l'autre élément vertébral (20), cette partie femelle recevant la partie mâle lorsque les éléments sont implantés sur leur vertèbre correspondante (1, 2).

7. Dispositif suivant la revendication 6, **caractérisé en ce que** la partie femelle (24) délimite un logement (25) de réception de la partie mâle (14), le fond (26) de ce logement portant, au moins en partie, la surface de guidage correspondante (26A).

8. Dispositif suivant la revendication 7, **caractérisé en ce que** la partie femelle (24) inclut une paroi médiale (27) et/ou une paroi latérale (28) de délimitation du logement (25), adaptées pour, lorsque les éléments (10, 20) sont implantés sur leur vertèbre correspondante (1, 2), retenir la partie mâle (14) suivant une direction médio-latérale.

9. Dispositif suivant l'une quelconque des revendications précédentes, **caractérisé en ce que** ledit axe de rotation est un axe géométrique (X-X).

10. Dispositif suivant l'une quelconque des revendications 1 à 8, **caractérisé en ce que** l'axe de rotation (X-X) est matérialisé par une tige (8) à même d'être inclinée par rapport à une direction médio-latérale au rachis, avec un angle maximal (α) inférieur à 10° environ, les surfaces de guidage (14A, 26A) correspondant sensiblement à des portions d'une sphère dont le centre (C) est situé sur l'axe de rotation (X-X).

11. Dispositif suivant l'une quelconque des revendications précédentes, **caractérisé en ce qu'**il comporte quatre éléments vertébraux associés par paires respectivement prévues pour être fixées sur les côtés gauche et droit des deux vertèbres (1, 2).

## Claims

1. Device (4) for the lateral stabilisation of the spine, said device being intended to reproduce, when in use, an articular intervertebral link and comprising at least two vertebral elements (10, 20) which are respectively adapted to be fixed onto one and the same lateral side of the body of at least two adjacent vertebrae (1, 2), **characterised in that** the two vertebral elements (10, 20) delimit respective surfaces (14A, 26A) for the relative guidance of the two elements, which surfaces are adapted to cooperate with one another, when the elements are implanted on their corresponding vertebra, as a result of complementarity of shapes in such a way that the said surfaces define an axis of rotation (X-X) about which the two elements are able to tilt in relation to one another and which extends both in a direction which is essentially medio-lateral to the spine and into the intervertebral discal space (7) separating the two adjacent vertebrae.

2. Device according to claim 1, **characterised in that** the guide surfaces (14A, 26A) define a permanent axis of rotation (X-X) when the vertebrae (1, 2) equipped with the two vertebral elements (10, 20) are displaced in relation to one another in accordance with a flexing/extending movement of the spine.

3. Device according to claim 1, **characterised in that** the guide surfaces (14A, 26A) define a number of instantaneous axes of rotation when the vertebrae (1, 2) equipped with the two vertebral elements (10, 20) are displaced in relation to one another in accordance with a flexing/extending movement of the spine.

4. Device according to any of the preceding claims, **characterised in that** the guide surfaces (14A, 26A) are also adapted to maintain a minimal clearance between the vertebrae (1, 2) in the longitudinal direction (3) of the spine.

5. Device according to claim 4, **characterised in that** the resultant (R) of contact between the two guide surfaces (14A, 26A) extends substantially parallel to the longitudinal direction (3) of the spine.

6. Device according to any of the preceding claims, **characterised in that** the guide surfaces (14A, 26A) are respectively provided by a male part (14) of one (10) of the two vertebral elements (10, 20) and by a female part (24) of the other vertebral element (20), the said female part receiving the male part when the elements are implanted on their corresponding vertebra (1, 2).

7. Device according to claim 6, **characterised in that** the female part (24) delimits a recess (25) for receiving the male part (14), the bottom (26) of the said recess carrying, at least partly, the corresponding guide surface (26A).

8. Device according to claim 7, **characterised in that** the female part (24) includes a medial wall (27) and/or a lateral wall (28) delimiting the recess (25), which walls are adapted to retain the male part (14) in a medio-lateral direction when the elements (10, 20) are implanted on their corresponding vertebra (1, 2).

9. Device according to any of the preceding claims, **characterised in that** the said axis of rotation is a geometrical axis (X-X).

10. Device according to any of claims 1 to 8, **characterised in that** the axis of rotation (X-X) is materialised by a rod (8) which is capable of being inclined in relation to a medio-lateral direction at the spine, with a maximum angle (α) of less than about 10°, the guide surfaces (14A, 26A) corresponding substantially to portions of a sphere, the centre of which is situated on the axis of rotation (X-X).

11. Device according to any of the preceding claims, **characterised in that** it comprises four vertebral elements associated in pairs which are respectively intended to be fixed onto the left and right sides of the two vertebrae (1, 2).

## Patentansprüche

1. Vorrichtung (4) zur seitlichen Stabilisierung der Wirbelsäule, die dazu bestimmt ist, nach ihrer Inbetriebnahme eine gelenkartige Verbindung zwischen den wirbeln nachzubilden, und die mindestens zwei wirbelseitige Elemente (10, 20) aufweist, die jeweils dazu geeignet sind, auf derselben Seite der Körper mindestens zweier benachbarter Wirbel (1, 2) befestigt zu werden, **dadurch gekennzeichnet, dass** die zwei wirbelseitigen Elemente (10, 20) jeweils Flächen (14A, 26A) begrenzen, die der relativen Führung der beiden Elemente dienen und derart ausgeführt sind, dass sie, nachdem die Elemente auf ihrem jeweiligen Wirbel implantiert wurden, aufgrund ihrer komplementären Formen derart zusammenwirken können, dass die Flächen eine Drehachse (X-X) festlegen, um welche die beiden Elemente gegeneinander eine Kippbewegung ausführen können und welche sich sowohl in eine Richtung erstreckt, die im Wesentlichen medio-lateral zur Wirbelsäule verläuft, als auch in den Bandscheibenbereich (7) zwischen den beiden benachbarten Wirbeln hinein.

2. Vorrichtung nach Anspruch 1, **dadurch gekennzeichnet, dass** die Führungsflächen (14A, 26A) dauerhaft eine Drehachse (X-X) festlegen, wenn die Wirbel (1, 2), die mit den zwei wirbelseitigen Elementen (10, 20) versehen sind, entsprechend einer Beugungs-/Dehnungsbewegung der Wirbelsäule gegeneinander bewegt werden.

3. Vorrichtung nach Anspruch 1, **dadurch gekennzeichnet, dass** die Führungsflächen (14A, 26A) mehrere, zeitweilig wechselnde Drehachsen festlegen, wenn die Wirbel (1, 2), die mit den zwei wirbelseitigen Elementen (10, 20) versehen sind, entsprechend einer Beugungs-/Dehnungsbewegung der Wirbelsäule gegeneinander bewegt werden.

4. Vorrichtung nach einem beliebigen der vorhergehenden Ansprüche, **dadurch gekennzeichnet, dass** die Führungsflächen (14A, 26A) weiterhin dazu geeignet sind, in Längsrichtung (3) der Wirbelsäule einen Mindestabstand zwischen den Wirbeln (1, 2) zu gewährleisten.

5. Vorrichtung nach Anspruch 4, **dadurch gekennzeichnet, dass** der Kontaktbereich (R), der sich zwischen den zwei Führungsflächen (14A, 26A) ergibt, sich im Wesentlichen parallel zur Längsrichtung (3) der Wirbelsäule erstreckt.

6. Vorrichtung nach einem beliebigen der vorhergehenden Ansprüche, **dadurch gekennzeichnet, dass** die Führungsflächen (14A, 26A) auf einem Steckelement (14) eines (10) der zwei wirbelseitigen Elemente (10, 20) beziehungsweise auf einem Steckbuchsenelement (24) des anderen wirbelseitigen Elements (20) vorgesehen sind, wobei dieses Steckbuchsenelement das Steckelement aufnimmt, wenn die Elemente auf ihren jeweiligen Wirbeln (1, 2) implantiert sind.

7. Vorrichtung nach Anspruch 6, **dadurch gekennzeichnet, dass** das Steckbuchsenelement (24) eine Aussparung (25) zur Aufnahme des Steckelements (14) begrenzt, wobei der Boden (26) dieser Aussparung zumindest teilweise die entsprechende Führungsfläche (26A) trägt.

8. Vorrichtung nach Anspruch 7, **dadurch gekennzeichnet, dass** das Steckbuchsenelement (24) eine Mittelwand (27) und/oder eine Seitenwand (28) zur Begrenzung der Aussparung (25) umfasst, die dazu geeignet ist, das Steckelement (14) in medio-lateraler Richtung zurückzuhalten, wenn die Elemente (10, 20) auf ihren jeweiligen Wirbeln (1, 2) implantiert sind.

9. Vorrichtung nach einem beliebigen der vorhergehenden Ansprüche, **dadurch gekennzeichnet, dass** es sich bei der Drehachse um eine geometrische Achse (X-X) handelt.

10. Vorrichtung nach einem beliebigen der vorhergehenden Ansprüche 1 bis 8, **dadurch gekennzeichnet, dass** die Drehachse (X-X) die materielle Form einer Stange (8) annimmt, die sich gegenüber einer Richtung, die medio-lateral zur Wirbelsäule verläuft, um einen Maximalwinkel (α) von weniger als ungefähr 10° neigen kann, wobei die Führungsflächen (14A, 26A) im Wesentlichen Abschnitten einer Kugel entsprechen, deren Mittelpunkt (C) auf der Drehachse (X-X) liegt.

11. Vorrichtung nach einem beliebigen der vorhergehenden Ansprüche, **dadurch gekennzeichnet, dass** sie vier wirbelseitige Elemente aufweist, die links und rechts der beiden Wirbel (1, 2) paarweise verbunden sind beziehungsweise dafür vorgesehen sind, dort befestigt zu werden.
